# FASCICULE DE BREVET EUROPEEN

(11) **EP 2 654 766 B1**
(45) Date de publication et mention de la délivrance du brevet: **10.10.2018**
(21) Numéro de dépôt: 11804575.6
(22) Date de dépôt: 22.12.2011
(51) Int. Cl.: A61K 36/54, A61K 8/97, A61P 17/00, A61P 17/10, A61Q 17/00, A61Q 19/00, A23L 19/00, A23L 33/105, A61K 9/00

(54) **EXTRAIT DE PULPE ET/OU DE PEAU D'AVOCAT RICHE EN POLYPHENOLS ET COMPOSITIONS COSMETIQUES, DERMATOLOGIQUES ET NUTRACEUTIQUES LE COMPRENANT**
EXTRAKTE AUS DEM FLEISCH UND/ODER HAUT DES AVOKADOS UND KOSMETISCHE SOWIE DERMATOLOGISCHE ODER NUTRAZEUTISCHE ZUSAMMENSETZUNGEN DIE DAS EXTRAKT BEINHALTEN.
EXTRACT OF THE PULP AND/OR PEEL OF AVOCADO RICH IN POLYPHENOLS AND COSMETIC, DERMATOLOGIC OR NUTRACEUTIC COMPOSITIONS COMPRISING THEREOF

(30) Priorité: 22.12.2010 FR 1061055
(43) Date de publication de la demande: 30.10.2013
(73) Titulaire: Laboratoires Expanscience, 92048 Paris La Défense Cedex (FR)
(72) Inventeur: SAUNOIS, Alex, 28100 Dreux (FR); BAUDOUIN, Caroline, 78120 Rambouillet (FR); LECLERE-BIENFAIT, Sophie, F-28100 Dreux (FR); GARNIER, Sébastien, 06650 Le Rouret (FR); MSIKA, Philippe, F-78000 Versailles (FR)
(74) Mandataire: Regimbeau
(86) Numéro de dépôt international: PCT/EP2011/073832
(87) Numéro de publication internationale: WO 2012/085224

(56) Documents cités:
- WO-A1-2005/105123
- WO-A1-2005/115421
- WO-A1-2010/100341
- FR-A1- 2 928 263
- FR-A1- 2 945 445
- DATABASE WPI Week 200430 Thomson Scientific, London, GB; AN 2004-323453 XP002671087, & JP 2004 123622 A (TOYO SHINYAKU KK) 22 avril 2004 (2004-04-22)
- DATABASE WPI Week 200430 Thomson Scientific, London, GB; AN 2004-322614 XP002671088, & JP 2004 115466 A (TOYO SHINYAKU KK) 15 avril 2004 (2004-04-15)
- WANG WEI ET AL: "Antioxidant capacities, procyanidins and pigments in avocados of different strains and cultivars", FOOD CHEMISTRY, vol. 122, no. 4, octobre 2010 (2010-10), pages 1193-1198, XP002654318, ISSN: 0308-8146
- PRABHA T N ET AL: "POLYPHENOLS OF AVOCADO (PERSEA AMERICANA) AND THEIR ENDOGENEOUS OXIDATION", JOURNAL OF FOOD SCIENCE AND TECHNOLOGY, SPRINGER (INDIA) PRIVATE LTD, INDIA, vol. 17, no. 5, 1 janvier 1980 (1980-01-01), pages 215-217, XP000923033, ISSN: 0022-1155
- DATABASE BIOSIS [Online] BIOSCIENCES INFORMATION SERVICE, PHILADELPHIA, PA, US; 2008, HIRASAWA MAKI ET AL: "Quantification and functional analysis of dietary fiber and polyphenols in avocado", XP002654320, Database accession no. PREV200800431658 & NIPPON SHOKUHIN KAGAKU KOGAKU KAISHI, vol. 55, no. 3, 2008, pages 95-101, ISSN: 1341-027X
- DATABASE WPI Week 200230 Thomson Scientific, London, GB; AN 2002-247100 XP002654317, "Agent for preventing and treating acne, comprises plant extract of shiitake mushroom, Centella asiatica, red pepper or meliloti, chlorella extract and placenta extract", & JP 2001 322943 A (KAO CORP) 20 novembre 2001 (2001-11-20)
- STAFFORD H A ET AL: "Procyanidins (Condensed Tannins) in Green Cell Suspension Cultures of Douglas Fir Compared with Those in Strawberry and Avocado Leaves by Means of C(18)-Reversed-phase Chromatography.", PLANT PHYSIOLOGY DEC 1980 LNKD- PUBMED:16661581, vol. 66, no. 6, décembre 1980 (1980-12), pages 1085-1090, XP002654319, ISSN: 0032-0889

## Description

L'invention se rapporte à un extrait de fruit d'avocat notamment de pulpe et/ou de peau d'avocat, riche en polyphénols, et aux compositions cosmétique, pharmaceutique, dermatologique, nutraceutique, comprenant un tel extrait et éventuellement un excipient approprié.

L'invention a également pour objet un procédé d'extraction d'un extrait d'avocat riche en polyphénols, ainsi que l'extrait susceptible d'être obtenu par ledit procédé.

L'invention concerne également une telle composition ou un tel extrait pour son utilisation dans la prévention ou le traitement des troubles ou pathologies de la peau, des muqueuses ou des phanères. L'invention concerne enfin un procédé de soin cosmétique de la peau, des phanères ou des muqueuses, en vue d'améliorer leur état ou leur aspect, consistant à administrer une telle composition ou un tel extrait.

L'avocatier (*Persea americana* ou *Persea gratissima*) appartient à la famille de Lauracées.

Il existe une grande diversité de variétés d'avocats. Parmi les plus répandues, on peut citer les variétés Hass, Fuerte, Ettinger, Bacon, Nabal, Ananheim, Lula, reed, Zutano, Quenn, Criola Selva, Mexicana Canta, Tegion Dschang, Hall, Booth, Peterson, Collinson Redn. On retiendra, particulièrement, les variétés Hass, Fuerte, Ettinger et Bacon, et plus avantageusement les variétés Hass et Fuerte. Le poids des fruits peut varier de 50 g à plus de 1 kg. Il est constitué de 10 à 20% par le noyau, 7 à 15% par les téguments, 65 à 80% par la pulpe. La peau est d'épaisseur, de texture et de couleur variables, lisse à rugueuse, en général verte mais pouvant devenir brune, violacée à noire.

La pulpe est de saveur et de couleur variables et est constituée en moyenne de :

**Tableau 1 : composition de la pulpe de l'avocat ; *Wei Wang, Food Chemistry, 122 : 1193 - 1198 (2010).**

| | |
|---|---|
| Eau | 70-85% |
| Protéines | 1,5-4,5% |
| Lipides | 12-23% |
| Sucres | 1,5-5% |
| Fibres | 1,1-1,6% |
| Polyphénols* | entre 0,06 et 0,5% dans la pulpe fraîche très variable = (variétés) |

La pulpe d'avocat est largement utilisée par les populations d'Amérique du sud pour faire briller les cheveux. Des applications topiques de pulpe sur la peau sont aussi très fréquentes pour son activité anti-oxydante et nourrissante, liées à la nature des lipides la composant (acides gras et fraction insaponifiable - vitamine E).

Les fruits, en particulier leur pulpe, sont utilisés couramment en médecine populaire sous les Tropiques. Ils sont très riches en vitamines A, B, C, D, E et K et recommandés aux diabétiques. La pulpe serait aussi ascaricide (et vermifuge), aphrodisiaque et tonique. Elle est utilisée en Amérique du sud en cataplasmes pour faire évoluer les furoncles et panaris.

Il a été démontré qu'une consommation élevée d'avocats avait un effet sur les niveaux de cholestérol sanguins. En particulier, après 7 jours d'un régime riche en avocats, des patients en hypercholestérolémie ont vu leur taux de cholestérol total diminuer de 17 %. Ces sujets ont également montré une diminution de 22 % des niveaux de (mauvais) cholestérol LDL et de triglycérides, et une augmentation de 11 % du (bon) cholestérol HDL. Ses acides gras mono insaturés sont excellents pour la santé cardiovasculaire et le bon cholestérol sanguin (HDL). De plus, l'avocat est une très bonne source de fibres (solubles et insolubles) et d'antioxydants.

On désigne sous le nom de « polyphénols », une très vaste famille de molécules, très répandue dans le monde végétal, caractérisées par la présence d'un ou plusieurs noyaux phénoliques, comportant notamment les sous-familles suivantes :
▪ acides phénoliques (cinnamique et benzoïque) ;
▪ flavonoïdes, pigments quasi-universels des végétaux, responsables de la coloration des fleurs, des fruits et parfois des feuilles. On trouvera dans cette famille, les anthocyanes (couleur bleue, à violette en passant par le rouge) ;
▪ quinones et anthraquinones et certaines formes condensées dérivées ; et
▪ des formes plus complexes et polymérisées : les tanins hydrolysables dérivés de l'acide gallique et ellagique ; des tanins condensés dérivés de l'acide catéchique, OPC ou Proanthocyanidols.
Ces molécules sont connues pour leurs nombreuses activités biologiques (anti-oxydantes, anti-inflammatoires, anti-bactériennes) et sont utilisées aussi bien dans les domaines de la pharmacie, de la cosmétique et de la nutrition/compléments alimentaires en fonction de certaines structures spécifiques.

Les polyphénols sont largement distribués dans de nombreuses familles du règne végétal supérieur car ils sont souvent indispensables à leur survie : protection face aux différentes agressions extérieures (UV, sécheresse, microorganismes...).

Parmi les polyphénols les plus répandus et exploités, on peut citer : les polyphénols de thé (*Camellia sinensis*), de raisin (*Vitis vinifera*), de cacao (*Cacao theobroma*)*.* La demande FR 2 928 263 décrit des compositions comprenant une association de polyphénols végétaux (les polyphénols de pomme sont particulièrement décrits) et de sucres végétaux. Les polyphénols recherchés sont les polyphénols polymériques, et en particulier les procyanidines et les prodelphinidines. L'avocat n'est pas cité comme source de sucres.

La demande internationale WO 2005/105123 décrit un médicament comprenant un extrait peptidique d'avocat et qui peut également comprendre une composition contenant du D-mannoheptulose et / ou du perséitol.

La demande internationale WO 2005/115421 décrit l'utilisation d'un composé comprenant du D-mannoheptulose et/ou du perséitol pour produire un médicament pour traiter et/ou prévenir des maladies liées à la modification d'une immunité innée et/ou acquise en augmentant la production de peptides antimicrobiens, de préférence hBD -2 sans induire de réactions inflammatoires, d'irritation ou d'intolérance. Ledit composé peut également comprendre un extrait peptidique d'avocat et/ou un extrait peptidique de lupin.

Les inventeurs ont découvert que les extraits d'avocat (*Persea gratissima ou americana*) riches en polyphénols présentent des propriétés cosmétiques, dermatologiques, pharmaceutiques ou nutraceutiques, en particulier des propriétés cosmétiques et dermatologiques, jamais décrites jusqu'à présent. En particulier, c'est la première fois que des extraits riches en polyphénols d'avocat sont utilisés en tant que tels, pour leurs propriétés spécifiques.

L'invention a pour objets en particulier:
- un extrait de fruit d'avocat riche en polyphénols, contenant au moins 10% en poids de polyphénols, exprimé en équivalent d'acide gallique par rapport à l'extrait sec obtenu, lesdits polyphénols étant des procyanidines, de l'acide caféique et des dérivés de l'acide caféique dans une proportion d'au moins 70% en poids exprimé en équivalent d'acide gallique par rapport à la teneur en poids totale de polyphénols. En particulier l'extrait contient 10 à 30% en poids, de polyphénols, exprimé en équivalent d'acide gallique par rapport au poids de l'extrait sec. Les proportions en procyanidines, de l'acide caféique et des dérivés de l'acide caféique dans lesdits polyphénols sont avantageusement d'au moins 80%, en poids exprimée en équivalent d'acide gallique par rapport à la teneur en poids totale de polyphénols.
Les procyanidines sont avantageusement choisies dans le groupe constitué par les procyanidines dimère de type B, les procyanidines trimère de type A et B et les procyanidines tétramère de type A et B.
Dans les polyphénols contenus dans cet extrait, on retrouve avantageusement au moins 30% en poids de procyanidines, exprimé en équivalent d'acide gallique par rapport à la teneur en poids totale de polyphénols.
Dans les polyphénols contenus dans cet extrait, on retrouve avantageusement l'acide caféique et ses dérivés, typiquement dans une proportion d'au moins 30% en poids, exprimé en équivalent d'acide gallique par rapport à la teneur en poids totale de polyphénols.
L'extrait comprend en outre au moins 10%, avantageusement de 10 à 60% de sucres d'avocat, lesdits sucres contenant au moins du D-mannoheptulose et/ou du perséitol ; les pourcentages étant exprimés en poids par rapport au poids total de l'extrait sec,
L'extrait est susceptible d'être obtenu par extraction de fruits de l'avocat qui ont été dans un premier temps séchés, dans des conditions douces, puis délipidés.
L'extrait est susceptible d'être obtenu par extraction solide-liquide d'une partie du fruit de l'avocat dans un solvant consistant en un mélange binaire de solvants du type eau et un solvant choisi parmi le glycérol ou un glycol tel que le propanediol, avantageusement dans des proportions comprises entre 0% à 100% d'eau par rapport aux autres solvants.
- une composition comprenant en tant qu'actif un extrait selon l'invention et, avantageusement un excipient approprié. En particulier, la composition est une composition cosmétique, pharmaceutique, dermatologique ou nutraceutique.
La composition comprend avantageusement en outre un autre actif, en particulier choisi dans le groupe constitué par que les émollients, les actifs hydratants, les kératorégulateurs, les kératolytiques, les agents cicatrisant et/ou restructurant de la barrière cutanée, les agonistes PPAR, RXR ou LXR, les agents sébo-régulateurs, les agents anti-irritants et/ou anti-inflammatoires et/ou apaisants, les agents anti-oxydants, les agents anti-âge, les agents dépigmentants ou hypopigmentants, les agents pigmentants les agents lipolytiques ou inhibiteurs de la lipogénèse ou encore les agents anti-cellulite ou amincissants, les filtres et écrans solaires minéraux ou organiques (pigmentaires ou ultrafins), les composés antifongiques, les conservateurs, les agents anti-bactériens, les pré et probiotiques, les antibiotiques, et les immunomodulateurs.
La composition comprend avantageusement comme autre actif un agent utile dans le traitement de l'acné, en particulier choisi dans le groupe constitué par les inhibiteurs de 5-alpha réductase, le zinc (et ses sels gluconate, salicylate et acide pyroglutamique), la spironolactone, l'acide linoléique, les antibiotiques, le péroxyde de benzoyle, l'acide azélaïque, la vitamine PP, la vitamine B3, les cyclines, les extraits des graines de citrouille, l'huile de pépins de courge et le sabal.
- un extrait ou une composition selon l'invention pour son utilisation en tant que composition pharmaceutique, dermatologique ou cosmétique ou en tant qu'aliment fonctionnel ; en particulier pour son utilisation dans la prévention et/ou le traitement des troubles ou pathologies de la peau et/ou des muqueuses et/ou des phanères, plus particulièrement pour son utilisation dans la prévention et/ou le traitement des réactions ou pathologies allergiques, inflammatoires, irritatives ou des troubles de la barrière ou de l'homéostasie de la peau, ou des troubles vasculaires, ou encore comme agent dépigmentant ou cicatrisant.
- un procédé de soin cosmétique de la peau et/ou des phanères et/ou des muqueuses, en vue d'améliorer leur état et/ou leur aspect, consistant à administrer une composition ou un extrait selon l'invention.

On entend par extrait d'avocat riche en polyphénols, un extrait obtenu par des procédés permettant de concentrer les polyphénols potentiellement présents dans l'avocat de manière à ce que cet extrait contienne au moins 10% en poids de polyphénols, exprimé en équivalent d'acide gallique par rapport au poids de l'extrait sec. Selon une variante avantageuse de l'invention, l'extrait contient 10% à 30% en poids de polyphénols, plus avantageusement 10% à 20% en poids de polyphénols, exprimé en équivalent d'acide gallique par rapport à l'extrait sec obtenu.

La teneur en extrait sec dans l'extrait selon l'invention, exprimée en poids par rapport au poids total de l'extrait, varie de 0,01 à 90%, avantageusement de 0,5 à 50%, plus avantageusement de 0,5 à 15%, encore plus avantageusement de 0,5 à 5%.

Dans les polyphénols contenus dans cet extrait, on retrouve des procyanidines, de l'acide caféique et des dérivés de l'acide caféique, en particulier des dérivés estérifiés, dans une proportion d'au moins 70%, avantageusement au moins 80%, plus avantageusement au moins 90%, encore plus avantageusement au moins 95%, en poids exprimée en équivalent d'acide gallique par rapport à la teneur en poids totale de polyphénols.

Les procyanidines sont des oligomères de catéchines/épicatéchine. La catéchine et l'épicatéchine sont respectivement les isomères optiques (+) et (-) de la molécule répondant à la formule (I) suivante :

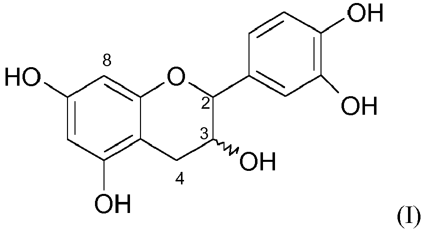

Les procyanidines sont avantageusement des procyanidines dimère de type B. Les procyanidines de type B-1, B-2, B-3 et B-4 sont des dimères constitués à partir de deux unités (+)-catéchine et (-)-épicatéchine, liées en C₄-C₈. Les procyanidines de type B-5, B-6, B-7 et B-8 sont des dimères constitués à partir de deux unités (+)-catéchine et (-)-épicatéchine, liées en C₄-C₆.

Les procyanidines sont avantageusement encore des procyanidines trimère de type A et B.

Les procyanidines sont avantageusement encore des procyanidines tétramère de type A et B.

Les procyanidines sont avantageusement encore des procyanidines oligomériques ou polymériques (nombre d'unités catéchine ou épicatéchine >4).

Dans les polyphénols contenus dans cet extrait, on retrouve avantageusement au moins 30%, plus avantageusement au moins 50% en poids de procyanidines, exprimé en équivalent d'acide gallique par rapport à la teneur en poids totale de polyphénols.

Dans les polyphénols contenus dans cet extrait, on retrouve également, avantageusement, l'acide caféique et ses dérivés dans une proportion d'au moins 20%, plus avantageusement au moins 30% en poids exprimé en équivalent d'acide gallique par rapport à la teneur en poids totale de polyphénols.

L'acide caféique répond à la formule (II) suivante :

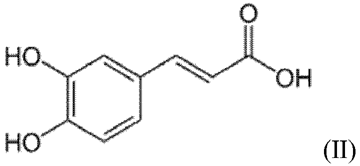

L'acide caféique et son dérivé, l'ester caféate de phénéthyle (ester de l'acide caféique et du 2-phényléthanol), sont présents dans de nombreuses plantes, et en particulier en grande quantité dans le café, dont il tire son nom. L'acide caféique peut être présent sous forme estérifiée, par exemple avec l'acide quinique avec lequel il forme l'acide chlorogénique. Parmi les autres formes estérifées, on peut citer l'acide caféoyltartrique (ou acide caftarique) formé avec l'acide tartrique, l'acide caféoylshikimique (formé avec l'acide shikimique), l'acide caféoylmalique (formé avec l'acide malique), ou encore estérifiée avec du glucose (caféoylglucose) ou sous forme d'amide avec la putrescine (caféoylputrescine).

D'autres polyphénols peuvent également être présents dans l'extrait selon l'invention, ils sont toutefois minoritaires. Leur teneur en poids, exprimée en équivalent d'acide gallique par rapport à la teneur en poids total de polyphénols, est avantageusement inférieure à 30%, plus avantageusement inférieure à 20%, encore plus avantageusement inférieure à 10%, encore plus avantageusement inférieure à 5%, encore plus avantageusement inférieure à 2%.

L'extrait contient au moins 10%, avantageusement de 10 à 60%, plus avantageusement de 10 à 30% de sucres d'avocat, typiquement lesdits sucres contenant au moins du D-mannoheptulose et/ou du perséitol (les % sont exprimés en poids par rapport au poids de l'extrait sec).

Dans le cadre de la présente invention, les termes « D-mannoheptulose » et « perséitol » couvrent également leurs dérivés chimiques.

Les sucres de l'avocat sont essentiellement le D-mannoheptulose, le perséitol, le saccharose, le glucose et le fructose. Des traces d'autres sucres peuvent être trouvées, mais leur teneur est inférieure à 5% en poids, avantageusement inférieure à 2% en poids, par rapport au poids total des sucres d'avocat.

Dans l'extrait selon l'invention, la proportion totale en D-mannoheptulose et/ou en perséitol dans les sucres d'avocats est avantageusement d'au moins 50% en poids, par rapport à la teneur en poids totale des sucres. Plus avantageusement, la proportion totale en D-mannoheptulose et/ou en perséitol dans les sucres d'avocat est de 50% à 98% en poids, plus avantageusement 55% à 95% en poids, encore plus avantageusement 60% à 90% en poids, par rapport à la teneur en poids totale des sucres.

Le ratio massique D-mannoheptulose : perséitol varie avantageusement de 1 : 10 à 10 : 1.

L'extrait selon l'invention contient avantageusement 0 à 50% en poids, plus avantageusement 0 à 20% en poids, encore plus avantageusement 0 à 10% en poids, de lipides d'avocats ; les % étant exprimés en poids par rapport au poids total de l'extrait sec.

L'extrait selon l'invention contient avantageusement 0 à 60% en poids, plus avantageusement 1 à 30% en poids, encore plus avantageusement 5 à 15% en poids, de protéines d'avocats ; les % étant exprimés en poids par rapport au poids total de l'extrait sec (dosage de Bradford).

L'extrait selon l'invention est obtenu par extraction d'une partie du fruit de l'avocat, préférentiellement de la pulpe et/ou de la peau. On utilise plus particulièrement le tourteau résiduel du pressage de la pulpe séchée d'avocat. Ladite pulpe a été préalablement séchée dans des conditions douces avant extraction des lipides.

Il est particulièrement avantageux d'utiliser des fruits (pulpe et/ou peau) d'avocats qui ont été dans un premier temps séchés, dans des conditions douces, puis délipidés. On a en effet constaté que dans de telles conditions, on assure une conservation optimale des polyphénols par inactivation de la polyphénol oxydase lors du séchage (on arrive même à obtenir des extraits ne contenant pas de polyphénol oxydase).

Avantageusement, cet extrait est obtenu par extraction solide-liquide, d'une partie du fruit de l'avocat dans un solvant consistant en un mélange binaire de solvants du type eau et un solvant choisi parmi le glycérol ou un glycol tel que le propanediol,. Les parties du fruit préférentiellement retenues sont la pulpe séchée et/ou déshuilée (tourteau) et/ou la peau, plus préférentiellement la pulpe séchée dans des conditions douces puis déshuilée.

Le solvant est avantageusement dans des proportions comprises entre 0% à 100% d'eau par rapport aux autres solvants.

Majoritairement, on utilise des mélanges binaires de solvants du type eau et un solvant choisi parmi le glycérol ou le propanediol.

Plus particulièrement, on introduira entre 0,1 et 50% en poids (exprimé par rapport au poids total avocat+solvant) de partie sèche d'avocat dans le solvant d'extraction, et préférentiellement 10% en poids.

En présence de glycérol, on choisira préférentiellement une concentration entre 40% et 90% de glycérol, et préférentiellement entre 60% et 80% (les % sont exprimés en poids de glycérol par rapport au poids total eau+glycérol).

En présence de glycol, et plus particulièrement de propanediol, on choisira préférentiellement une proportion entre 40% et 80%, et avantageusement entre 50% et 60% de propanediol par rapport à l'eau (les % sont exprimés en poids de propanediol par rapport au poids total eau+propanediol).

La température d'extraction est avantageusement comprise entre 4°C et 100°C, et préférentiellement entre 10°C et 60°C, et plus particulièrement entre 15°C et 30°C.

La durée d'extraction varie avantageusement de 30 minutes à 4 heures, plus particulièrement de 30 minutes à 2 heures.

Ces différentes extractions peuvent être suivies d'étapes de purification par ultrafiltration (seuil de coupure de 10 kDa par exemple), ou diafiltration et/ou nanofiltration (seuil de coupure de 200 Da par exemple), permettant de concentrer les polyphénols au dépend des sucres (dont les sucres spécifiques de l'avocat en C7, mannoheptulose et perséitol).

L'extrait obtenu suite à ces étapes de purification comprend avantageusement 10% à 30% en poids de polyphénols, encore plus avantageusement 10% à 20% en poids de polyphénols, exprimé en équivalent d'acide gallique par rapport au poids de l'extrait sec.

La teneur en sucres dans l'extrait obtenu suite à ces étapes de purification est avantageusement comprise entre 5 et 20% en poids, par rapport au poids de l'extrait sec.

Les sucres présents dans cet extrait comprennent avantageusement 20 à 60% de D-mannoheptulose et/ou de perséitol, plus avantageusement 30 à 60% de D-mannoheptulose et/ou de perséitol, exprimé en poids par rapport au poids total de sucres.

L'extrait obtenu pourra se présenter sous forme liquide, mais également pourra être séché selon les méthodes connues de l'Homme de l'art, l'atomisation ou la lyophilisation par exemple avec ou sans support telle la maltodextrine.

L'invention a également pour objet un procédé de préparation d'un extrait d'avocat riche en polyphénols comprenant les étapes successives suivantes :
(a) dispersion en phase liquide dans un solvant adapté du fruit (avantageusement pulpe et/ou peau) de l'avocat, et avantageusement de la pulpe déshuilée séchée (tourteau) ;
(b) soumission du mélange obtenu suite à l'étape (a) à une extraction sous agitation dans un solvant aqueux et/ou alcoolique et/ou glycolique et/ou glycérolique, avantageusement un mélange d'eau et d'un solvant choisi dans le groupe constitué par l'éthanol, le glycérol, un glycol (avantageusement le propanediol) et leurs mélanges ; dans une proportion avantageusement de 60% de ces solvants dans l'eau ;
(c) centrifugation de l'extrait obtenu suite à l'étape (b) puis filtration ou filtration directement ;
(d) le cas échéant, soumission de l'extrait obtenu suite à l'étape (c) à une étape d'ultrafiltration et/ou de diafiltration et/ou de nanofiltration ;
(e) suite à l'étape (c) ou (d), récupération de l'extrait riche en polyphénols ; et
(f) séchage éventuel de l'extrait obtenu suite à l'étape (e) sur un support ou non.

L'extrait est avantageusement utilisé en tant qu'agent actif dans une composition cosmétique, pharmaceutique, dermatologique ou nutraceutique, qui peut comprendre un ou plusieurs excipients appropriés. La composition peut en outre comprendre au moins un autre composé actif en plus de l'extrait d'avocat riche en polyphénols. Cet autre composé peut être choisi parmi tous les composés et leurs équivalents fonctionnels, énoncés ci-dessous.

Cet autre composé peut être en particulier choisi parmi des actifs classiquement utilisés en dermatologie, en pharmaceutique, en cosmétique ou en nutraceutique et connus de l'homme de l'art tels que les émollients, les actifs hydratants, les kératorégulateurs, les kératolytiques, les agents cicatrisant et/ou restructurant de la barrière cutanée, les agonistes PPAR, RXR ou LXR, les agents sébo-régulateurs, les agents anti-irritants et/ou anti-inflammatoires et/ou apaisants, les agents anti-oxydants, les agents anti-âge, les agents dépigmentants ou hypopigmentants, les agents pigmentants, les agents lipolytiques ou inhibiteurs de la lipogénèse ou encore les agents anti-cellulite ou amincissants, les filtres et écrans solaires minéraux ou organiques (pigmentaires ou ultrafins), les composés antifongiques, les conservateurs, les agents anti-bactériens, les pré et probiotiques, les antibiotiques, les immunomodulateurs.

Plus particulièrement, les agents cicatrisants et/ou restructurants de la barrière cutanée pouvant être utilisés en association sont avantageusement le panthénol (vitamine B5), l'arabinogalactane, l'oxyde de zinc, les céramides, le cholestérol, le squalane et les phospholipides.

Les agents sébo-régulateurs pouvant être utilisés en association sont avantageusement choisis dans le groupe constitué par les inhibiteurs de 5-alpha réductase, tels que le zinc et ses dérivés (sels gluconate, salicylate et acide pyroglutamique).

L'agent anti-inflammatoire et/ou anti-irritant et/ou apaisant peut être l'arabinogalactane.

Parmi les agents hypopigmentants ou dépigmentants, on peut notamment citer la N-undecylenoyl-L-phénylalanine (Sepiwhite®).

Les actifs protecteurs solaires pouvant être utilisés en association sont avantageusement des filtres ou écrans solaires UVB et/ou UVA ; tels les écrans ou filtres minéraux et/ou organiques connus de l'homme du métier qui adaptera leur choix et leurs concentrations en fonction du degré de protection recherché.

Les conservateurs pouvant être utilisés en association sont par exemple ceux généralement utilisés en cosmétique ou en nutraceutique, les molécules à activité anti-bactérienne (pseudo-conservateurs) tels que les dérivés capryliques comme par exemple le capryloyl glycine et le glycéryl caprylate ; l'hexanediol, le sodium levulinate, et les dérivés de zinc et de cuivre (gluconate et PCA).

Parmi les actifs recommandés en association avec l'extrait selon l'invention, on peut citer les extraits végétaux, en particulier :
- Les huiles végétales telles que les huiles de Soja et/ou l'huile de Colza, l'huile d'avocat (WO2004/012496, WO2004/012752, WO2004/016106, WO2007/057439), l'huile de Lupin, avantageusement l'huile de Lupin blanc doux (WO 98/47479), ou un mélange de ces huiles ;
- l'oléodistillat ou les concentrats d'huile végétale ou animale, notamment de tournesol, plus avantageusement des concentrats de Tournesol linoléiques, tels que l'huile de tournesol concentrée en insaponifiables (Soline®) (cf. la demande internationale WO 01/21150) commercialisée par les Laboratoires Expanscience, les huiles concentrées en insaponifiable du type huile d'avocat, de colza, de maïs ou de palme, utiles notamment pour leur activité hydratante et/ou émolliente, cicatrisante et/ou restructurante de la barrière cutanée, anti-inflammatoire et/ou anti-irritante et/ou apaisante ;
- les insaponifiables de végétaux ou d'huile végétale, avantageusement des furanes d'avocat (Avocadofurane®), pouvant être obtenus par le procédé décrit dans la demande internationale WO 01/21605, les insaponifiables d'Avocat et/ou de Soja, plus particulièrement un mélange d'insaponifiables d'Avocat furaniques et d'insaponifiables de Soja, avantageusement dans un rapport respectif d'environ 1/3-2/3 (tel que Piasclédine®), les insaponifiables de soja (tels qu'obtenus selon le procédé décrit dans la demande internationale WO01/51596), les insaponifiables stéroliques (typiquement des insaponifiables dont la teneur en stérols, en méthylstérols et en alcools triterpèniques est comprise entre 20 et 95 % en poids, de préférence 45-65 % en poids, par rapport au poids total de l'insaponifiable), les phytostérols, les esters de stérols et les dérivés vitaminiques, utiles notamment pour leur activité cicatrisante et/ou restructurante de la barrière cutanée, anti-âge, anti-inflammatoire ;
- les peptides ou complexes d'acides aminés végétaux, en particulier les peptides d'avocat (tel que ceux décrits dans la demande internationale WO2005/105123), les peptides de lupin (tels que ceux obtenus selon le procédé décrit dans la demande WO2005/102259), les peptides de quinoa (tels que ceux décrits dans la demande internationale WO2008/080974), les peptides de Maca tels que ceux décrits dans la demande internationale WO2004/112742), les peptides de soja fermentés ou non, les peptides de riz (tels que ceux décrits dans la demande internationale WO 2008/009709), utiles notamment pour leur activité hydratante et/ou émolliente (avocat), kératorégulatrice (lupin, quinoa), cicatrisante et/ou restructurante de la barrière cutanée (maca, quinoa, soja), anti-inflammatoire et/ou anti-irritante et/ou apaisante (lupin, quinoa), antioxydante (avocat), anti-âge (lupin, maca), pigmentante (riz) ;
- les sucres de végétaux, en particulier les sucres d'avocat (tels que ceux décrits dans la demande WO2005/115421), utiles notamment pour leurs propriétés kératorégulatrice, cicatrisante et/ou restructurante de la barrière cutanée, anti-inflammatoire et/ou anti-irritante et/ou apaisante ;
- le butyle avocadate (5 alpha Avocuta®), inhibiteur de la 5-alpha réductase (voir WO 01/52837 et WO 02/06205), typiquement régulateur de la sécrétion séborrhée se trouvant augmentée dans l'acné ou les pellicules ;
- les extraits riches en polyphénols, et plus particulièrement les extrait de parties aériennes de Gynandropsis gynandra (FR 1 061 051) et les extraits de feuilles de Maca (FR 1 061 047) ;
- le lupéol (FR 2 822 821, FR 2 857 596) utile notamment pour favoriser la cicatrisation,
- un extrait total de lupin (tel que ceux décrits dans la demande internationale WO2005/102259), particulièrement adapté pour le traitement des irritations ;
- un extrait de graines *d'Acacia macrostachya* (FR 0958525), un extrait de feuilles de *maca* (FR 1 061 047), un extrait de graines de *Schizandra sphenanthera* (FR 0955343 et FR 0955344) et degraines de *Vigna unguiculata* (FR 0958529).
Parmi les actifs recommandés en association avec l'extrait selon l'invention, on peut citer les oxazolines, en particulier celles choisies dans le groupe constitué par la 2-undécyl-4-hydroxyméthyl-4-méthyl-1,3-oxazoline, la 2-undécyl-4,4-diméthyl-1,3-oxazoline, la (E)-4,4-diméthyl-2-heptadéc-8-ényl-1,3-oxazoline, la 4-hydroxyméthyl-4-méthyl-2-heptadécyl-1,3-oxazoline, la (E)-4-hydroxyméthyl-4-méthyl-2-heptadéc-8-ényl-1,3-oxazoline, la 2-undécyl-4-éthyl-4-hydroxyméthyl-1,3-oxazoline, de préférence la 2-undécyl-4,4-diméthyl-1,3-oxazoline (OX-100 ou Cyclocéramide® ; WO2004050052, WO2004050079, et WO2004112741). Elles sont particulièrement utiles pour leur activité anti-inflammatoire et/ou anti-irritante et/ou apaisante, antioxydante, dépigmentant, immunomodulatrice.

Parmi les actifs recommandés en association avec l'extrait selon l'invention, on peut citer les inhibiteurs de 5-alpha réductase, tels que le butyle avocadate (5 alpha Avocuta®).

Toutes ces associations comprennent au moins un autre composé actif, en plus de l'extrait d'avocat riche en polyphénols, et peuvent comprendre deux, trois, quatre ou plus de composés actifs tels que décrits précédemment.

La composition selon l'invention peut être formulée sous la forme de différentes préparations adaptées à une administration topique, à une administration orale, rectale, vaginale, nasale, auriculaire ou bronchique, ainsi qu'à une administration parentérale.

Selon une première variante, les différentes préparations sont adaptées à l'administration topique et incluent notamment les crèmes, les émulsions, les laits, les pommades, les lotions, les huiles, les solutions aqueuses ou hydro-alcooliques ou glycoliques, les poudres, les patchs, les sprays, les shampooings, les vernis ou tout autre produit pour application externe.

Selon une deuxième variante, les différentes préparations sont adaptées à une administration orale ; l'extrait d'avocat riche en polyphénols pouvant entrer soit dans un complément alimentaire soit dans une composition nutraceutique. Le complément alimentaire peut se présenter sous forme de l'extrait d'avocat riche en polyphénols en tant que tel ou bien sous forme de gélules ou de capsules molles de gélatine ou végétales dans le cadre de la présente invention. Ledit complément alimentaire peut alors contenir de 10 à 100% en poids de l'extrait d'avocat riche en polyphénols.

La composition de la présente invention peut être incorporée directement et sans autre modification dans les nutraceutiques, les produits diététiques notamment hyperprotéinés ou les breuvages et ce grâce à des techniques comme le mixage, l'infusion, l'injection, le mélange, l'absorption, le pétrissage et la pulvérisation.

Les modes d'administration, les posologies et les formes galéniques optimales des composés et compositions selon l'invention peuvent être déterminés selon les critères généralement pris en compte dans l'établissement d'un traitement pharmaceutique, en particulier dermatologique, cosmétique ou vétérinaire adapté à un patient ou à un animal comme par exemple l'âge ou le poids corporel du patient ou de l'animal, la gravité de son état général, la tolérance au traitement, les effets secondaires constatés, le type de peau. En fonction du type d'administration souhaité, la composition et/ou les composés actifs selon l'invention peuvent en outre comprendre au moins un excipient pharmaceutiquement acceptable, notamment dermatologiquement acceptable, ou un excipient cosmétiquement ou nutraceutiquement acceptable. Selon la première variante, on utilise un excipient adapté pour une administration par voie topique externe. La composition selon la présente invention peut en outre comprendre au moins un adjuvant pharmaceutique ou cosmétique connu de l'homme du métier, choisi parmi les épaississants, les conservateurs, les parfums, les colorants, des filtres chimiques ou minéraux, les agents hydratants, les eaux thermales, etc.

La composition comprenant un extrait d'avocat riche en polyphénols ayant les spécifications indiquées est particulièrement destinée à une utilisation cosmétique, pharmaceutique ou dermatologique. La composition sera avantageusement formulée sous la forme d'une préparation adaptée à une administration topique.

L'invention a également pour objet l'utilisation d'un extrait d'avocat riche en polyphénols, pour la fabrication d'une composition cosmétique, pharmaceutique, dermatologique, d'une composition nutraceutique ou d'un aliment fonctionnel.

Avantageusement, la composition ou l'extrait selon la présente invention est utilisée dans la prévention et/ou le traitement des troubles ou pathologies de la peau et/ou des muqueuses et/ou des phanères.

En particulier, la composition ou l'extrait selon l'invention est destiné à la prévention et/ou au traitement des réactions ou pathologies allergiques, inflammatoires, irritatives ou des troubles de la barrière ou de l'homéostasie de la peau, des phanères (cheveux et ongles) et/ou des muqueuses (gencives, parodontes, muqueuses génitales) immature(s), normale(s) ou mature(s)/âgée(s).

On entend par troubles de la barrière de la peau, des phanères et/ou des muqueuses les troubles intervenant au niveau de la couche extérieure de l'épiderme.

On entend par troubles de l'homéostasie de la peau, des phanères et/ou des muqueuses les troubles résultant des processus de renouvellement et d'équilibre des cellules telles que le psoriasis, la dermite du siège, la dermatite atopique, la peau sèche (xérose), la peau déshydratée et la peau photosensibilisée.

Avantageusement, la composition ou l'extrait selon l'invention peut être utilisé(e) pour la prévention et/ou le traitement des réactions, troubles ou pathologies :
- de la peau, telles que, la rosacée ou érythrocouperose, le psoriasis, les troubles vasculaires, la dermite du siège, la dermatite atopique, l'eczéma, la dermatite de contact, la dermatite irritative, la dermatite allergique, la dermite séborrhéique (croûte de lait), la peau sensible, la peau réactive, le prurit, la peau sèche (xérose), la peau déshydratée, la peau avec rougeur, l'érythème cutanée, la peau âgée ou photoâgée, la peau photosensibilisée, la peau pigmentée (mélasma, pigmentation post-inflammatoire), la peau avec cellulite, la peau relâchée, la peau avec vergetures, les dartres, les gerçures, les piqûres, les crevasses en particulier des seins, les coups de soleil, les inflammations dus aux rayons de toutes sortes, les irritations par agents chimiques, physiques (par exemple contrainte de tension pour les femmes enceintes), bactériologiques, fongiques ou viraux, parasitaires (poux, gale, teigne, acariens, dermatophytes), radiologiques ou par déficit de l'immunité innée (peptides antimicrobiens) ou acquise (cellulaire, humorales, cytokines), et/ou
- des muqueuses telles que les gencives et parodontes pouvant présenter des gingivites (gencives sensibles des nouveaux nés, problèmes d'hygiène, dus au tabagisme ou autres), des parodontopathies, ou des muqueuses génitales pouvant présenter des irritations des sphères génitales males ou femelles externes ou internes et/ou
- des phanères tels que les ongles (ongles cassants, fragiles ...) et des cheveux (alopécie, pellicules, hirsutismes, dermites séborrhéiques, folliculites) immatures, normaux ou matures, présentant en particulier des troubles du cuir chevelu tels que les alopécies (ou pelade) androgénétiques, aiguës, localisées, cicatricielles, congénitales, occipitales du nourrisson, aerata, dues à la chimiothérapie/radiothérapie ou encore l'effluvium télogène, l'effluvium anagène, la dystrophie pilaire, la trichotillomanie, la teigne ou les pellicules grasses ou sèches.

La composition ou l'extrait selon la présente invention est également avantageusement utilisé(e) dans la prévention et/ou le traitement des troubles vasculaires, et peut être avantageusement utilisé(e) dans la dépigmentation par diminution de la mélanine et/ou inhibition de la tyrosinase, ou comme agent cicatrisant.

L'invention concerne également un procédé de soin cosmétique de la peau et/ou des phanères et/ou des muqueuses, en vue d'améliorer leur état et/ou leur aspect, comprenant l'administration ou consistant à administrer une composition cosmétique ou un extrait selon la présente invention.

Dans un mode de réalisation du procédé cosmétique selon l'invention, la peau et/ou les phanères et/ou les muqueuses visées sont avantageusement celles qui sont sensibles, irritées, agressées par l'environnement (UV, pollution), en particulier les peaux sensibles.

Le procédé cosmétique selon l'invention est également caractérisé en ce que la composition ou l'extrait est utilisé(e) en tant que produit hydratant, ou en tant que produit anti-âge chronologique ou photo-induit dans la prévention du vieillissement, et du vieillissement photo-induit, ou en tant que produit amincissant et/ou anticellulite, ou encore en tant que produit anti-âge ou produit anti-tâche.

On entend par produit hydratant, un produit permettant notamment, de prévenir et/ou traiter les troubles de la barrière ou de l'homéostasie de la peau, des phanères et/ou des muqueuses.

On entend par produit anti-tâche, un produit permettant de réduire les tâches de pigmentation de la peau et/ou des phanères et/ou des muqueuses.

On entend par produit amincissant et/ou anticellulite, un produit permettant d'améliorer la fermeté, l'élasticité ou la tonicité de la peau, et/ou de lutter contre l'accumulation du tissu adipeux et la peau avec cellulite.

### Exemple 1 - comparatif

La pulpe d'avocat séchée et déshuilée (tourteau) est mise en suspension sous agitation à 10% dans un mélange éthanol/eau 60/40 p/p pendant 1 h à température ambiante. La matière sèche résiduelle est séparée de la phase liquide soit par filtration, décantation ou centrifugation, et la phase liquide ainsi obtenue peut être filtrée à l'aide de filtres de porosité adaptée afin d'obtenir une solution limpide. L'extrait obtenu présente les caractéristiques suivantes :
▪ Extrait sec : 2,56%
▪ Sucres totaux (glucose, fructose, mannoheptulose, perséitol - HPLC) : 19,5% /sec
▪ Polyphénols totaux (Folin - Ciocalteu, éq. acide gallique) : 14% / sec
▪ Protéines (Bradford) : 6,25% / sec.

Cet extrait présente une activité anti-radicalaire, anti-DPPH « in tubo », pour laquelle la concentration inhibitrice 50 (IC50) a pu être déterminée et est de 111 µg d'extrait sec, ce qui représente 10,6 µg de polyphénols dans le milieu réactionnel.

### Exemple 2

La pulpe d'avocat séchée et déshuilée (tourteau) est mise en suspension sous agitation à 10% dans un mélange glycérol/eau 60/40 p/p pendant 1 h à température ambiante. La matière sèche résiduelle est séparée de la phase liquide soit par filtration, décantation ou centrifugation, et la phase liquide ainsi obtenue peut être filtrée à l'aide de filtres de porosité adaptée afin d'obtenir une solution limpide. L'extrait obtenu présente les caractéristiques suivantes :
▪ Extrait 3,45%
▪ Sucres totaux (glucose, fructose, mannoheptulose, perséitol - HPLC) : 19% /sec
▪ Polyphénols totaux (Folin - Ciocalteu, éq. acide gallique) : 12% / sec
▪ Protéines (Bradford) : 9% / sec.

Cet extrait présente une activité anti-radicalaire, anti-DPPH « in tubo », pour laquelle la concentration inhibitrice 50 (IC50) a pu être déterminée et est de 84 µg d'extrait sec, ce qui représente 11,85 µg de polyphénols dans le milieu réactionnel.

### Exemple 3

La pulpe d'avocat séchée et déshuilée (tourteau) est mise en suspension sous agitation à 10% dans un mélange propanediol/eau 60/40 p/p pendant 1 h à température ambiante. La matière sèche résiduelle est séparée de la phase liquide soit par filtration, décantation ou centrifugation, et la phase liquide ainsi obtenue peut être filtrée à l'aide de filtres de porosité adaptée afin d'obtenir une solution limpide. L'extrait obtenu présente les caractéristiques suivantes :
▪ Extrait 2,74%
▪ Sucres totaux (glucose, fructose, mannoheptulose, perséitol - HPLC) : 22% /sec
▪ Polyphénols totaux (Folin - Ciocalteu, éq. Acide gallique) : 16% / sec
▪ Protéines (Bradford) : 7% / sec.

Cet extrait présente une activité anti-radicalaire, anti-DPPH « in tubo », pour laquelle la concentration inhibitrice 50 (IC50) a pu être déterminée et est de 119,5 µg d'extrait sec, ce qui représente 18,5 µg de polyphénols dans le milieu réactionnel.

### Exemple 4 : Compositions pour application par voie topique

Les inventeurs présentent ci-dessous plusieurs compositions pour application par voie topique. Les extraits d'avocat riches en polyphénols (*Persea gratissima et americana*) peuvent être incorporés à divers produits cosmétiques, tels que des eaux nettoyantes, des émulsions huile dans eau, des émulsions eau dans huile, des huiles, des laits, des lotions, des shampooings, des produits moussants et sprays, dont les compositions sont présentées ci-dessous.

**EAU NETTOYANTE PEAU SENSIBLE**

| **Nom commercial ou INCI** | **%** |
|---|---|
| CAPRYLOYL GLYCINE | De 0 à 1 % |
| LESSIVE SOUDE | De 0 à 1 % |
| SEQUESTRANT | De 0 à 1 % |
| BUTYLENE GLYCOL | De 1 à 5 % |
| BETA CAROTENE | De 0 à 2 % |
| **Extrait d'avocat riche en polyphénols** | De 0,01 à 10% |
| CONSERVATEURS | De 0 à 1 % |
| PEG-32 | De 1 à 5 % |
| PEG-7 PALMCOCOATE | De 1 à 5 % |
| GLUCONATE ZINC | De 0 à 1 % |
| ACIDE CITRIQUE | De 0 à 1 % |
| EAU PURIFIÉE | QSP 100 % |
| PARFUM | De 0 à 1 % |
| POLOXAMER 184 | De 1 à 5 % |

**EMULSION ANTI-AGE**

| **Nom commercial ou INCI** | **%** |
|---|---|
| ISOPARAFFINE LIQUIDE | De 5 à 20 % |
| STEARATE D'ISOCETYLE | De 5 à 20 % |
| HYDROXYSTEARATE AL - MG | De 5 à 20 % |
| ABIL WE 09 | De 1 à 5 % |
| GLYCEROL | De 1 à 5 % |
| HUILE VASELINE | De 1 à 5 % |
| ZINC OXYDE MICRONISE | De 1 à 5 % |
| BUTYLENE GLYCOL | De 1 à 5 % |
| RETINOL | De 0 à 1% |
| VITAMINE C | De 0 à 5% |
| **Extrait d'avocat riche en polyphénols** | De 0,01 à 10 % |
| ISONONYL ISONONANOAT | De 1 à 5 % |
| CIRE D'ABEILLE | De 1 à 5 % |
| TARTRATE DE SODIUM | De 1 à 5 % |
| CHLORURE DE SODIUM | De 0 à 5 % |
| GLYCINE | De 1 à 5 % |
| CONSERVATEURS | De 0 à 1% |
| CHOLESTEROL | De 0 à 1 % |
| PHYTOSPHINGOSINE | De 0 à 1 % |
| ACIDE TARTRIQUE | De 0 à 1 % |
| EAU PURIFIÉE | QSP 100 % |

**LAIT pour PEAU SECHE, ATOPIQUE**

| **Matière première / Nom commercial ou INCI** | **%** |
|---|---|
| HUILE AMANDE DOUCE | De 1 à 5 % |
| HUILE MAIS | De 1 à 5 % |
| ACIDE STEARIQUE | De 1 à 5 % |
| ALCOOL CETYLIQUE C16 C18 | De 0 à 1 % |
| ANTIMOUSSE 70414 | De 0 à 1 % |
| ALCOOL LAURIQUE 11OE | De 1 à 5 % |
| MONOLAURATE PEG 300 | De 0 à 1 % |
| MONOLEATE DE GLYCEROL | De 0 à 1 % |
| MONOSTEARATE DE GLYCEROL | De 1 à 5 % |
| VITAMINE B12 | De 0 à 5% |
| **Extrait d'avocat riche en polyphénols** | De 0,1 à 10 % |
| CONSERVATEURS | De 0 à 1 % |
| ACIDE CITRIQUE | De 0 à 1 % |
| CITRATE TRISODIQUE | De 0 à 1 % |
| EAU PURIFIEE | QSP 100 % |
| PARFUM | De 0 à 1 % |
| HUILE ARACHIDE | De 1 à 5 % |
| HUILE PALMISTE HYDROGENEE | De 1 à 5 % |

**CREME LAVANTE PURIFIANTE**

| **Matière première / Nom commercial ou INCI** | **%** |
|---|---|
| EAU PURIFIEE | QSP 100 % |
| ARLATONE | De 10 à 30 % |
| COCOGLUCOSIDE | De 5 à 20 % |
| HYDROXYPROPYL GUAR | De 1 à 5 % |
| CAPRYLOYL GLYCINE | De 0 à 2 % |
| CONSERVATEURS | De 0 à 2 % |
| PARFUM | De 0 à 1 % |
| ACIDE CITRIQUE | De 0 à 1% |
| ZINC PCA | De 0 à 1 % |
| **Extrait d'avocat riche en polyphénols** | De 0,01 à 10 % |

**SHAMPOOING ANTIPELLICULAIRE**

| **Matière première / Nom commercial ou INCI** | **%** |
|---|---|
| EAU PURIFIÉE | QSP 100 % |
| LAUROAMPHOACETATE | De 5 à 20 % |
| COCOGLUCOSIDE | De 5 à 20 % |
| DISTEARATE DE PEG 6000 | De 1 à 5 % |
| CONSERVATEURS | De 0 à 2 % |
| VITAMINE F | De 0 à 5 % |
| PIROCTONE OLAMINE | De 0 à 2 % |
| **Extrait d'avocat riche en polyphénols** | De 0,01 à 10 % |
| ZINC PYRITHIONE | De 0 à 1 % |
| AJUSTEUR pH | De 0 à 1 % |
| SEQUESTRANT | De 0 à 1 % |
| PARFUM | De 0 à 1 % |

### Exemple 5 : Compositions pour administration par voie orale

Les extraits d'avocat sont intégrés à des compositions orales, dans des compositions permettant l'administration de 50 mg à 200 mg d'extrait d'avocat riche en polyphénols par jour.

### 1/ Composition anti-vergetures sous forme de capsules molles

| | |
|---|---|
| **- Extrait d'avocat riche en polyphénols** | 30 mg |
| - Huile d'Awara | 60 mg |
| - Huile de colza riche en insaponifiable | 300 mg |
| - Vitamine du groupe B (B1, B2, B3, B5, B6, B9, B12), | QSP 100% des AJR |
| - Tocotriénols | QSP 50 % AJR |
| - Vitamine E | |
| - Cire d'abeille | |
| - Lécithine de soja | |
| - Gélatine alimentaire | |
| - Glycérine | QSP 1 capsule molle |

Cette composition est administrée de 4 à 6 capsules de 500 mg par jour.

### 2/ Comprimés anti-chute cheveux

| | |
|---|---|
| - **Extrait d'avocat riche en polyphénols** | 25 mg |
| - Extraits de céréales (blé, sarrasin, millet, épeautre) | |
| riche en acides aminés soufrés | 200 mg |
| - Vitamine C | QSP 50 % des AJR |
| - Glycosaminoglycanes issus de cartilages de poissons | 200 mg |
| - Glucidex IT 19 (agent de compression) | QSP |
| | 1 comprimé de 800 mg. |

Cette composition est administrée de 5 à 8 comprimés par jour.

### 3/ Exemples en stick poudre amincissant

| | |
|---|---|
| - **Extrait d'avocat riche en polyphénols** | 100 mg |
| - Extrait de thé riche en polyphénols | 100 mg |
| - Extrait de raisin riche en OPC | 50 mg |
| - Bétaglucanes d'origine végétale | 100 mg |
| - Gomme xanthane | 1 mg |
| - ascorbate de sodium | 0,3 mg |
| - maltodextrine | QSP 5 g |

Cette composition est administrée 2 fois par jour.

### Exemple 6 : Activités biologiques

### 1. Effet anti-inflammatoire

### a. Activité anti-inflammatoire sur des kératinocytes

Dans la peau, le kératinocyte est l'une des premières cellules participant à l'initiation de la réaction inflammatoire en réponse à une agression de l'environnement.

Le kératinocyte « agressé » va alors relarguer des cytokines qui vont induire une cascade de réactions impliquant le système immunitaire.

### ✔ Matériel et méthodes :

Des kératinocytes humains (lignée NCTC-2544) ont été pré-incubés ou non (contrôle) par l'extrait d'avocat concentré en polyphénols (AV) à 0,005% et 0,01% (p/v de matière active) ou les molécules de référence anti-inflammatoires (dexaméthasone à 10⁻⁷M ; indométhacine à 10⁻⁶M) pendant 24 heures. Les cellules ont ensuite été traitées par le PMA à 0,1 µg/ml (Phorbol Myristate Acetate) pendant 24 heures, toujours en présence de AV ou des références.

A l'issue du traitement, les quantités d'IL8 (interleukine 8) et de PGE2 (prostaglandine E2) sécrétées ont été mesurées par ELISA dans les surnageants de culture.

Les résultats ont été analysés statistiquement par un test t de Student.

### ✔ Résultats et conclusion :

AV a fortement et significativement inhibé la production des médiateurs inflammatoires IL8 et PGE2 stimulée par le PMA dans des kératinocytes (tableau 1).

L'extrait AV possède donc une activité anti-inflammatoire.

**Tableau 1 : Production d'IL8 et de PGE2 par des kératinocytes**

| | **IL8** (ng/ml) | Inhibition | |
|---|---|---|---|
| Cellules contrôles | 0,1 ± 0,0 | | |
| PMA 0,1 µg/ml | 50,1 ± 1,8 | | |
| Dexaméthasone 10-7M | 7,4 ± 0,8 | 85% | p<0,001 |
| **AV 0,005%** | **24,2 ± 1,4** | **52%** | p<0,001 |
| **AV 0,01%** | **13,7 ± 0,6** | **73%** | p<0,001 |
| | | | |

| | **PGE2** (ng/ml) | Inhibition | |
|---|---|---|---|
| Cellules contrôles | 0,039 ± 0,0 | | |
| PMA 0,1 µg/ml | 138,4 ± 10,6 | | |
| Indométhacine 10-6M | 0,039 ± 0,0 | 100% | p<0,001 |
| **AV 0,005%** | **32,3 ± 3,1** | **77%** | p<0,001 |
| **AV 0,01%** | **10,1 ± 0,3** | **93%** | p<0,001 |

### b. Inhibition de la production de leukotriène B4 par des neutrophiles

Le leukotriène B4 (LTB4) est un médiateur inflammatoire lipidique issu de la voie de l'acide arachidonique.

Le LTB4 est produit et libéré en quantité importante par le polynucléaire neutrophile humain *via* l'activation de l'enzyme 5 lipoxygénase ; il joue un rôle essentiel dans le développement des réactions inflammatoires cutanées.

### ✔ Matériel et méthodes :

Des neutrophiles humains ont été pré-incubés pendant 15 minutes en présence de l'extrait d'avocat concentré en polyphénols (AV) à 0,005% ; 0,01% et 0,1% (p/v de matière active).

Les cellules ont ensuite été stimulées par ajout de zymosan opsonisé à 1 mg/ml.

Après 10 minutes d'incubation, le leukotriène B4 (LTB4) libéré par les cellules a été dosé dans les surnageants de cellules par une technique ELISA.

Les résultats ont été analysés statistiquement par un test t de Student.

### ✔ Résultats et conclusion :

AV a significativement inhibé la production de LTB4 induite par le zymosan opsonisé sur des neutrophiles (tableau 2).

Ainsi, l'extrait AV module l'inflammation induite par le neutrophile et LTB4.

**Tableau 2 : Production de leukotriène B4 par des neutrophiles**

| | **LTB4** (pg/ml) | Inhibition | |
|---|---|---|---|
| Cellules contrôles | 31 ± 2,8 | | |
| Cellules stimulées | 2900 ± 496,7 | | |
| **AV 0,005%** | **2210 ± 779,3** | **-24%** | **ns** |
| **AV 0,01%** | **1737,5 ± 197,4** | **-40%** | **p<0,01** |
| **AV 0,1%** | **1925 ± 434,9** | **-34%** | **p<0,05** |

### c. Inhibition de la libération d'histamine

Les mastocytes jouent un rôle important dans les réactions allergiques et inflammatoires ; largement distribué chez l'homme dans les tissus conjonctifs, tels que la peau, le mastocyte séreux représente la cellule sentinelle de la réponse inflammatoire locale. La substance P, neuromédiateur peptidique, agit sur le mastocyte cutané en induisant une libération rapide d'histamine préformée dans les granules de stockage ; ce mécanisme d'inflammation neurogène est impliqué dans diverses pathologies cutanées.

### ✔ Matériel et méthode :

Des mastocytes ont été pré-incubés pendant 30 minutes en présence de l'extrait d'avocat concentré en polyphenols (AV) à 0,01% (p/v de matière active) ou de calcium à 10 mM (inhibiteur de référence de la libération d'histamine).

Les mastocytes ont ensuite été stimulés par la substance P à 10 µM pendant 15 minutes. A la fin de l'incubation, l'histamine libérée a été quantifiée par ELISA.

Les résultats ont été analysés statistiquement par un test t de Student.

### ✔ Résultats et conclusion :

AV a significativement inhibé la libération d'histamine par des mastocytes stimulés par la substance P (tableau 3).

L'extrait AV module l'inflammation neurogène notamment liée à l'histamine.

**Tableau 3 : Libération d'histamine par des mastocytes**

| | **Histamine** (ng/ml) | Inhibition | |
|---|---|---|---|
| Contrôle | 20,1 ± 1,9 | | |
| Substance P | 142,5 ± 9,6 | | |
| Calcium 10 mM | 21,8 ± 1,0 | -85% | p<0,01 |
| **AV 0,01%** | **112,5 ± 9,6** | **-21%** | **p<0,01** |

### 2. Activité dépigmentante

L'effet dépigmentant de l'extrait AV a été étudié dans deux modèles distincts : par évaluation de la production de mélanine dans des mélanocytes ; et par évaluation de l'activité enzymatique de la tyrosinase, enzyme clé impliquée dans la synthèse de mélanine.

### a. Inhibition de la mélanogénèse

### ✔ Matériel et méthode :

Des mélanocytes épidermiques humains normaux ont été cultivés en présence de NDP-MSH à 10⁻⁷M (analogue de l'α-MSH ; induction de la mélanogénèse) et de l'extrait d'avocat concentré en polyphenols (AV) à 0,001% et 0,005% (p/v de matière active) ou d'acide kojique à 0,25mM (référence).

Après 240 heures d'incubation, la mélanine a été extraite des cellules et quantifiée par spectrophotométrie.

Les résultats ont été analysés statistiquement par un test t de Student.

### ✔ Résultats et conclusion :

AV a significativement inhibé la production de mélanine par les mélanocytes stimulés par le NDP-MSH (tableau 4).

AV présente donc un effet dépigmentant.

**Tableau 4 : Production de mélanine par des mélanocytes**

| | **Mélanine** (µg/ml) | | |
|---|---|---|---|
| Cellules contrôles | 24,7 ± 0,0 | | |
| Témoin stimulé (NDP-MSH) | 30,4 ± 0,9 | +19% | p<0,01 |
| Référence (acide kojique) | 9,6 ± 0,6 | -32% | p<0,001 |
| **AV 0,001%** | **26,2 ± 0,6** | **-14%** | **p<0,05** |
| **AV 0,005%** | **25,7 ± 0,5** | **-15%** | **p<0,05** |

### b. Inhibition de l'activité tyrosinase

### ✔ Matériel et méthode :

L'extrait d'avocat concentré en polyphenols (AV), ainsi que l'acide kojique (référence) à différentes concentrations ont été pré-incubés en présence de tyrosinase extraite de mélanocytes humains pendant 10 minutes à froid.

Le substrat de l'enzyme, L-DOPA à 2mM, a ensuite été ajouté.

Après 1 heure d'incubation à 37°C, l'activité enzymatique a été évaluée par mesure spectrophotométrique.

Les résultats ont été analysés statistiquement par un test t de Student.

### ✔ Résultats et conclusion :

AV a significativement inhibé l'activité enzymatique de la tyrosinase (Tableau 5). Ce résultat confirme l'effet dépigmentant de cet extrait.

**Tableau 5 : Activité de la tyrosinase humaine**

| | **Tyrosinase** (U/ml) | Inhibition | |
|---|---|---|---|
| Témoin | 182,7 | | |
| Acide kojique 0,0625mM | 166,8 | -9% | p<0,05 |
| Acide kojique 0,25mM | 152,4 | -17% | p<0,001 |
| **AV 0,001%** | **165,5** | **-9%** | **p<0,05** |
| **AV 0,005%** | **164,7** | **-10%** | **p<0,05** |
| **AV 0,01%** | **162,5** | **-11%** | **p<0,01** |
| **AV 0,05%** | **148,2** | -**19%** | **p<0,001** |
| **AV 0,1%** | **142,4** | **-22%** | **p<0,001** |

### 3. Activité cicatrisante : stimulation des marqueurs de la ré-épithélialisation

Le mécanisme de cicatrisation en réponse à une blessure implique pour la réparation épidermique un processus de ré-épithélialisation.

La ré-épithélialisation cutanée consiste en la régénération par les kératinocytes d'un épithélium organisé, pavimenteux, stratifié, kératinisé qui recouvre la plaie et qui reforme une barrière protectrice.

Le mécanisme de ré-épithélialisation se fait selon trois étapes : migration des kératinocytes, prolifération et maturation de l'épiderme.

L'effet de l'extrait AV a été étudié sur l'expression génique de marqueurs impliqués dans l'étape de migration des kératinocytes au cours de ce processus de ré-épithélialisation : la laminine 5 et la MMP9 (matrix metalloprotease 9).

### ✔ Matériel et méthode :

Des kératinocytes humains normaux ont été incubés pendant 48 heures en présence de l'extrait d'avocat concentré en polyphénols (AV) à 0,001% et 0,005% (p/v de matière active) ou de TGFβ1 à 5 ng/ml (référence).

L'expression génique de la MMP9 et la laminine 5 (sous-unité γ2) a été étudiée par RT-PCR en temps réel.

Les résultats ont été analysés statistiquement par une ANOVA à un facteur suivie d'un test de Dunnett : ns p>0,05 (non significatif) ; *p<0,05 ; ***p<0,001.

### ✔ Résultats et conclusion :

AV a significativement augmenté l'expression des marqueurs de la migration des kératinocytes (tableau 6).

Cet extrait présente donc un effet activateur de la ré-épithélialisation, en faveur d'un effet réparateur, pro-cicatrisant.

**Tableau 6 : Expression génique des marqueurs de migration kératinocytaires (Quantité Relative)**

| | **Laminine 5** | | **MMP9** | |
|---|---|---|---|---|
| Cellules contrôles | 1,00 | | 1,00 | |
| Référence (TGFβ1) | 11,33 | +1033% *** | 19,84 | +1884% *** |
| **AV 0,001%** | **1,62** | **+62% *** | **3,81** | **+281% ***** |
| **AV 0,005%** | **1,51** | **+51% *** | **9,17** | **+817% ***** |

## Revendications

1. Extrait de fruit d'avocat riche en polyphénols, contenant au moins 10% en poids de polyphénols et avantageusement entre 10 à 30% en poids de polyphénols, exprimé en équivalent d'acide gallique par rapport à l'extrait sec obtenu, lesdits polyphénols contenant un mélange de procyanidines, de l'acide caféique et des dérivés de l'acide caféique, dans une proportion d'au moins 70% en poids, exprimé en équivalent d'acide gallique par rapport à la teneur en poids totale de polyphénols, et comprenant en outre au moins 10%, avantageusement de 10 à 60% de sucres d'avocat, lesdits sucres contenant au moins du D-mannoheptulose et/ou du perséitol, les pourcentages étant exprimés en poids par rapport au poids de l'extrait sec, **caractérisé en ce qu'**il est susceptible d'être obtenu par extraction de fruits de l'avocat qui ont été dans un premier temps séchés, dans des conditions douces, puis délipidés, et **en ce qu'**il est susceptible d'être obtenu par extraction solide-liquide d'une partie du fruit de l'avocat dans un solvant consistant en un mélange binaire de solvants du type eau et un solvant choisi parmi le glycérol ou un glycol tel que le propanediol.

2. Extrait selon la revendication 1, **caractérisé en ce que** les proportions en procyanidines, avantageusement choisies dans le groupe constitué par les procyanidines dimère de type B, les procyanidines trimère de type A et B et les procyanidines tétramère de type A et B, de l'acide caféique et des dérivés de l'acide caféique dans lesdits polyphénols sont d'au moins 80% en poids, exprimé en équivalent d'acide gallique par rapport à la teneur en poids totale de polyphénols.

3. Extrait selon la revendication 1 ou 2, **caractérisé en ce que** dans les polyphénols contenus dans cet extrait, on retrouve au moins 30% de procyanidines, exprimé en équivalent d'acide gallique par rapport à la teneur en poids totale de polyphénols.

4. Extrait selon l'une quelconque des revendications précédentes, **caractérisé en ce que** dans les polyphénols contenus dans cet extrait, on retrouve l'acide caféique et ses dérivés dans une proportion d'au moins 30% en poids, exprimé en équivalent d'acide gallique par rapport à la teneur en poids totale de polyphénols.

5. Composition comprenant en tant qu'actif un extrait tel qu'il est défini dans l'une quelconque des revendications 1 à 4 et un excipient approprié, **caractérisée en ce qu'**il s'agit d'une composition cosmétique, pharmaceutique, dermatologique ou nutraceutique.

6. Composition selon la revendication 5, comprenant en outre un autre actif, en particulier choisi dans le groupe constitué par que les émollients, les actifs hydratants, les kératorégulateurs, les kératolytiques, les agents cicatrisants et/ou restructurants de la barrière cutanée, les agents sébo-régulateurs, les agents anti-irritants et/ou anti-inflammatoires et/ou apaisants, les agents anti-oxydants, les agents anti-âge, les agents dépigmentants ou hypopigmentants, les agents pigmentants, les agents lipolytiques ou inhibiteurs de la lipogénèse ou encore les agents anti-cellulite ou amincissants, les filtres et écrans solaires minéraux ou organiques (pigmentaires ou ultrafins), les composés antifongiques, les conservateurs, les agents anti-bactériens, les pré et probiotiques, les antibiotiques, et les immunomodulateurs.

7. Composition selon la revendication 5 ou 6, **caractérisée en ce que** l'autre actif est choisi parmi :
• les agents cicatrisants et/ou restructurants de la barrière cutanée, de préférence le panthénol, l'arabinogalactane, l'oxyde de zinc, les céramides, le cholestérol, le squalane et les phospholipides,
• les agents sébo-régulateurs, de préférence choisis parmi les inhibiteurs de 5-alpha réductase, les dérivés de zinc,
• les agents anti-inflammatoires et/ou anti-irritants et/ou apaisants, de préférence l'arabinogalactane,
• les agents hypopigmentants ou dépigmentants, de préférence la N-undecylenoyl-L-phénylalanine.
• les filtres et écrans solaires minéraux ou organiques, de préférence les filtres et écrans solaires UVB et/ou UVA, et
• les conservateurs de préférence choisis parmi le capryloyl glycine, le glycéryl caprylate et l'hexanediol.

8. Composition selon l'une quelconque des revendications 5 à 7, comprenant en outre au moins un autre actif choisi dans le groupe constitué parmi :
• les huiles végétales, de préférence l'huile de soja, l'huile de colza, l'huile d'avocat, l'huile de lupin, et avantageusement l'huile de lupin blanc doux, ou un mélange de ces huiles,
• les oléodistillats ou les concentrats d'huile végétale ou animale, de préférence de tournesol, d'avocat, de colza, de maïs et de palme et avantageusement concentrés en insaponifiables,
• les insaponifiables de végétaux ou d'huile végétale, de préférence les insaponifiables d'avocats, les insaponifiables de soja ou leurs mélanges, avantageusement des furanes d'avocat, et en particulier, un mélange d'insaponifiables d'Avocat furaniques et d'insaponifiables de Soja dans un rapport respectif d'environ 1/3-2/3, les insaponifiables stéroliques, les phytostérols, les esters de stérols et les dérivés vitaminiques,
• les peptides ou complexes d'acides aminés végétaux, de préférence les peptides d'avocat, les peptides de lupin, les peptides de quinoa, les peptides de Maca, les peptides de soja fermentés ou non, les peptides de riz,
• les sucres de végétaux, de préférence les sucres d'avocat,
• le butyle avocadate,
• les extraits riches en polyphénols, de préférence les extraits de parties aériennes *de Gynandropsis gynandra* et les extraits de feuilles de Maca,
• le lupéol,
• un extrait total de lupin,
• un extrait de graines *d'Acacia macrostachya,* un extrait de feuilles de *maca,* un extrait de *Schizandra sphnanthera* et un extrait de graines de *Vigna unguiculata,*
• les oxazolines, de préférence la 2-undécyl-4-hydroxyméthyl-4-méthyl-1,3-oxazoline, la 2-undécyl-4,4-diméthyl-1,3-oxazoline, la (E)-4,4-diméthyl-2-heptadéc-8-ényl-1,3-oxazoline, la 4-hydroxyméthyl-4-méthyl-2-heptadécyl-1,3-oxazoline, la (E)-4-hydroxyméthyl-4-méthyl-2-heptadéc-8-ényl-1,3-oxazoline, la 2-undécyl-4-éthyl-4-hydroxyméthyl-1,3-oxazoline et la 2-undécyl-4,4-diméthyl-1,3-oxazoline, et
leurs mélanges.

9. Composition selon l'une quelconque des revendications 5 à 8, **caractérisée en ce qu'**elle est formulée pour être administrée par voie topique ou orale.

10. Composition selon l'une quelconque des revendications 5 à 9 ou extrait tel qu'il est défini dans l'une quelconque des revendications 1 à 4 pour son utilisation dans la prévention et/ou le traitement des troubles ou pathologies de la peau et/ou des muqueuses et/ou des phanères, avantageusement des réactions ou pathologies allergiques, inflammatoires, irritatives ou des troubles de la barrière ou de l'homéostasie de la peau, ou des troubles vasculaires ou encore comme agent dépigmentant et cicatrisant.

11. Procédé de soin cosmétique de la peau et/ou des phanères et/ou des muqueuses, avantageusement des peaux sensibles, en vue d'améliorer leur état et/ou leur aspect, en particulier en vue de favoriser leur hydratation, ou encore d'améliorer la fermeté, l'élasticité ou la tonicité de la peau et de lutter contre l'accumulation du tissu adipeux et la peau avec cellulite, ou encore de réduire les tâches de pigmentation, ou encore pour prévenir et/ou traiter le vieillissement, comprenant l'administration d'une composition cosmétique selon l'une quelconque des revendications 5 à 9 ou d'un extrait tel que défini selon l'une quelconque des revendications 1 à 4.

## Patentansprüche

1. An Polyphenolen reicher Avocadofruchtextrakt, der mindestens 10 Gew.-% Polyphenole und vorteilhafterweise zwischen 10 und 30 Gew.-% Polyphenole enthält, als Gallussäureäquivalent bezogen auf den gewonnenen Trockenextrakt ausgedrückt, wobei die Polyphenole eine Mischung von Procyanidinen, Kaffeesäure und Kaffeesäurederivate in einem Anteil von mindestens 70 Gew.-% enthalten, als Gallussäureäquivalent bezogen auf den Gesamtpolyphenolgehalt ausgedrückt, und weiterhin mindestens 10 %, vorteilhafterweise 10 bis 60 % Avocadozucker, wobei der Zucker mindestens D-Mannoheptulose und/oder Perseitol enthält, wobei die Prozentsätze als Gewicht bezogen auf das Gewicht des Trockenextrakts ausgedrückt sind, **dadurch gekennzeichnet, dass** er durch Extraktion von Avocadofrüchten, die anfänglich unter milden Bedingungen getrocknet und dann delipidiert wurden, gewonnen wird, und dass er durch Fest-Flüssig-Extraktion eines Teils der Avocadofrucht in einem Lösungsmittel gewonnen wird, das aus einer binären Mischung von Lösungsmitteln vom Wassertyp und einem unter Glyzerol oder einem Glykol, wie Propandiol, ausgewählt ist, besteht.

2. Extrakt nach Anspruch 1, **dadurch gekennzeichnet, dass** die Anteile an Procyanidinen, die vorteilhaft aus der Gruppe bestehend aus Procyanidindimeren vom Typ B, Procyanidintrimeren vom Typ A und B und Procyanidintetrameren vom Typ A und B, Kaffeesäure und Kaffeesäurederivaten in den Polyphenolen ausgewählt sind, mindestens 80 Gew.-%, als Gallussäureäquivalent bezogen auf den Gesamtgewichtsgehalt an Polyphenolen ausgedrückt, betragen.

3. Extrakt nach Anspruch 1 oder 2, **dadurch gekennzeichnet, dass** die in diesem Extrakt enthaltenen Polyphenole mindestens 30 % Procyanidine, als Gallussäureäquivalent bezogen auf den Gesamtpolyphenolgehalt ausgedrückt, enthalten.

4. Extrakt nach einem beliebigen der vorstehenden Ansprüche, **dadurch gekennzeichnet, dass** die in diesem Extrakt enthaltenen Polyphenole Kaffeesäure und ihre Derivate mit einem Anteil von mindestens 30 Gew.-%, als Gallussäureäquivalent bezogen auf den Gesamtpolyphenolgehalt ausgedrückt, enthalten.

5. Zusammensetzung, die als aktiven Bestandteil einen Extrakt wie in einem beliebigen der vorstehenden Ansprüche 1 bis 4 definiert und einen geeigneten Hilfsstoff enthält, **dadurch gekennzeichnet, dass** es sich um eine kosmetische, pharmazeutische, dermatologische oder nutrazeutische Zusammensetzung handelt.

6. Zusammensetzung nach Anspruch 5, die ferner einen anderen Wirkstoff umfasst, der insbesondere aus der Gruppe bestehend aus Weichmachern, feuchtigkeitsspendenden Wirkstoffen, Keratoregulatoren, Keratolytika, Wirkstoffen zur Wundheilung und/oder Restrukturierung der Hautbarriere, Seboregulatoren, reizlindernden und/oder entzündungshemmenden und/oder beruhigenden Mitteln, Antioxydantien, Anti-Aging-Stoffen, Depigmentier- oder Hypopigmentierungsmitteln, Pigmentierungsmitteln, Lipolytischen Mitteln oder Lipogenese-Hemmern oder auch Anti-Cellulite- oder Körperpflegemitteln, mineralischen oder organischen Sonnenschutzmitteln und Filtern (Pigment- oder ultrafein), antimykotischen Mitteln, Konservierungsmitteln, antibakteriellen Mitteln, Prä- und Probiotika, Antibiotika und Immunmodulatoren ausgewählt ist.

7. Verfahren nach Anspruch 5 oder 6, **dadurch gekennzeichnet, dass** der andere Wirkstoff ausgewählt ist unter:
• Wirkstoffen zur Wundheilung und/oder Restrukturierung der Hautbarriere, vorzugsweise Panthenol, Arabinogalactan, Zinkoxid, Ceramiden, Cholesterin, Squalan und Phospholipiden,
• seboregulatorischen Mitteln, vorzugsweise ausgewählt unter 5-Alpha-Reduktase-Hemmern, Zink-Derivaten,
• entzündungshemmenden und/oder reizlindernden und/oder beruhigenden Mitteln, vorzugsweise Arabinogalactan,
• hypopigmentierenden oder depigmentierenden Mitteln, vorzugsweise N- Undecylenoyl-L-phenylalanin,
• mineralischen oder organischen Filtern und Sonnenschutzmitteln, vorzugsweise UVB- und/oder UVA-Filtern und Sonnenschutzmitteln, und
• Konservierungsmitteln, die vorzugsweise unter Capryloylglycin, Glycerylcaprylat und Hexandiol ausgewählt sind.

8. Zusammensetzung nach einem beliebigen der vorstehenden Ansprüche 5 bis 7, die ferner zumindest einen weiteren Aktivstoff enthält, der ausgewählt ist aus der Gruppe bestehend aus Folgenden:
• Pflanzenöle, vorzugsweise Sojaöl, Rapsöl, Avocadoöl, Lupinenöl und vorteilhafterweise süßes weißes Lupinenöl oder eine Mischung dieser Öle,
• Oleodestillate oder Konzentrate von pflanzlichen oder tierischen Ölen, vorzugsweise Sonnenblumen, Avocado, Raps, Mais und Palmen und vorteilhafterweise in Unverseifbaren konzentriert,
• Unverseifbare aus Pflanzen oder Pflanzenöl, vorzugsweise Avocado Unverseifbare, Soja Unverseifbare oder Mischungen davon, vorzugsweise Avocadofurane Unverseifbare, insbesondere eine Mischung aus Avocadofuran Unverseifbaren und Soja Unverseifbaren im jeweiligen Verhältnis von etwa 1/3 - 2/3, Sterol Unverseifbare, Phytosterine, Sterolester und Vitamin-Derivate,
• pflanzliche Aminosäurepeptide oder -komplexe, vorzugsweise Avocadopeptide, Lupinpeptide, Quinoapeptide, Macapeptide, fermentierte oder unfermentierte Sojapeptide, Reispeptide,
• Pflanzenzucker, vorzugsweise Avocadozucker,
• Avocadobutyl,
• Extrakte, die reich an Polyphenolen sind, vorzugsweise Extrakte aus oberirdischen Pflanzenteilen de *Gynandropsis gynandra* und Extrakte aus Maca-Blättern,
• Lupeol,
• ein totaler Lupinenextrakt
• ein Samenextrakt aus & *Acacia macrostachya,* ein Extrakt aus maca Blättern, ein Extrakt aus *Schizandra sphnanthera* und ein Samenextrakt aus *Vigna unguiculata,*
• Oxazoline, vorzugsweise 2-Undecyl-4-hydroxymethyl-4-methyl-1,3-oxazolin, 2-Undecyl-4,4-dimethyl-1,3-oxazolin, (E)-4,4-dimethyl-2-heptadec-8-enyl-1,3-oxazolin, 4-Hydroxymethyl-4-methyl-2-heptadecyl-1,3-Oxazolin, (E)-4-Hydroxymethyl-4-methyl-2-heptadec-8-enyl-1,3-oxazolin, 2-Undecyl-4-ethyl-4-hydroxymethyl-1,3-oxazolin und 2-Undecyl-4,4-dimethyl-1,3-oxazolin, und
Mischungen davon.

9. Zusammensetzung nach einem beliebigen der vorstehenden Ansprüche 5 bis 8, **dadurch gekennzeichnet, dass** sie zur topischen oder oralen Verabreichung formuliert ist.

10. Zusammensetzung nach einem beliebigen der vorstehenden Ansprüche 5 bis 9 oder Extrakt nach einem beliebigen der vorstehenden Ansprüche 1 bis 4 zur Verwendung bei der Vorbeugung und/oder Behandlung von Hauterkrankungen oder -pathologien und/oder der Schleimhäute und/oder Hautanhangsgebilde, vorteilhafterweise der allergischen, entzündlichen, irritativen Reaktionen oder Pathologien oder Störungen der Hautbarriere oder Homöostase oder Gefäßerkrankungen oder auch als Depigmentierungs- und Wundheilmittel.

11. Verfahren zur kosmetischen Pflege der Haut und/oder der Hautanhangsgebilde und/oder der Schleimhäute, vorteilhaft der empfindlichen Haut, um ihren Zustand und/oder ihr Aussehen zu verbessern, insbesondere, um ihre Durchfeuchtung zu unterstützen, oder um die Festigkeit, die Elastizität oder die Spannkraft der Haut zu verbessern und die Ansammlung von Fettgewebe und der Haut mit Cellulite zu bekämpfen, oder zur Verringerung von Pigmentflecken oder zur Vorbeugung und/oder Behandlung von Hautalterung, umfassend die Verabreichung einer kosmetischen Zusammensetzung nach einem beliebigen der vorstehenden Ansprüche 5 bis 9 oder eines Extrakts nach einem beliebigen der vorstehenden Ansprüche 1 bis 4.

## Claims

1. A polyphenol-rich extract of avocado fruit, containing at least 10% by weight of polyphenols and advantageously between 10 and 30% by weight of polyphenols, expressed in gallic acid equivalents in relation to the dry extract obtained, said polyphenols containing a mixture of procyanidins, caffeic acid and derivatives of caffeic acid, in a proportion of at least 70% by weight, expressed in gallic acid equivalents in relation to the total polyphenols content by weight, and further including at least 10%, advantageously from 10 to 60% avocado sugars, said sugars containing at least D-mannoheptulose and/or perseitol, the percentages being expressed by weight in relation to the weight of the dry extract, **characterized in that** it is obtainable by extraction of avocado fruits that first have been dried, under gentle conditions, then delipidated, and **in that** it is obtainable by solid-liquid extraction of a portion of the avocado fruit in a solvent consisting of a binary mixture of solvents of the water type and a solvent selected from glycerol or a glycol such as propanediol.

2. The extract of claim 1, **characterized in that** the proportions of procyanidins, advantageously selected from the group consisting of B-type procyanidin dimers, A- and B-type procyanidin trimers and A- and B-type procyanidin tetramers, of caffeic acid and of derivatives of caffeic acid in said polyphenols are at least 80% by weight, expressed in gallic acid equivalents in relation to the total polyphenols content by weight.

3. The extract of claim 1 or claim 2, **characterized in that** the polyphenols contained in this extract contain at least 30% of procyanidins, expressed in gallic acid equivalents in relation to the total polyphenols content by weight.

4. The extract of any one of the preceding claims, **characterized in that** the polyphenols contained in this extract contain caffeic acid and derivatives thereof in a proportion of at least 30% by weight, expressed in gallic acid equivalents in relation to the total polyphenols content by weight.

5. A composition including as active agent an extract as defined in any one of claims 1 to 4 and a suitable carrier, **characterized in that** it is a cosmetic, pharmaceutical, dermatological or nutraceutical composition.

6. The composition of claim 5, further including another active agent, in particular selected from the group consisting of emollients, moisturizing active agents, keratoregulators, keratolytics, agents that heal and/or restructure the cutaneous barrier, sebum-regulating agents, anti-irritation and/or anti-inflammatory and/or soothing agents, antioxidant agents, anti-aging agents, depigmenting or hypopigmenting agents, pigmenting agents, lipolytic or lipogenesis inhibitor agents or anti-cellulitis or slimming agents, organic or mineral sun screens and filters (pigmentary or ultrafine), antifungal compounds, preservatives, antibacterial agents, prebiotics and probiotics, antibiotics, and immunomodulators.

7. The composition of claim 5 or claim 6, **characterized in that** the other active agent is selected from:
• agents that heal and/or restructure the cutaneous barrier, preferably panthenol, arabinogalactan, zinc oxide, ceramides, cholesterol, squalane and phospholipids,
• sebum-regulating agents, preferably selected from 5-alpha reductase inhibitors and zinc derivatives,
• anti-inflammatory and/or anti-irritation and/or soothing agents, preferably arabinogalactan,
• hypopigmenting or depigmenting agents, preferably N-undecylenoyl-L-phenylalanine,
• organic or mineral sun screens and filters, preferably UVB and/or UVA sun screens and filters, and
• preservatives preferably selected from capryloyl glycine, glyceryl caprylate and hexanediol.

8. The composition of any one of claims 5 to 7, further including at least one other active agent selected from the group consisting of:
• plant oils, preferably soy oil, rapeseed oil, avocado oil, lupin oil, and advantageously sweet white lupin oil, or a mixture of these oils,
• oleodistillates or concentrates of plant or animal oil, preferably of sunflower, avocado, rapeseed, corn and palm and advantageously concentrated in unsaponifiables,
• unsaponifiables of plants or plant oil, preferably avocado unsaponifiables, soy unsaponifiables or mixtures thereof, advantageously of avocado furans, and in particular, a mixture of avocado furanic unsaponifiables and soy unsaponifiables in a respective ratio of approximately 1/3-2/3, sterolic unsaponifiables, phytosterols, esters of sterols and vitamin derivatives,
• peptides or complexes of plant amino acids, preferably avocado peptides, lupin peptides, quinoa peptides, maca peptides, fermented or non-fermented soy peptides, rice peptides,
• plant sugars, preferably avocado sugars,
• butyl avocadate,
• polyphenol-rich extracts, preferably extracts of the above-ground parts of *Gynandropsis gynandra* and extracts of maca leaves,
• lupeol,
• a total lupin extract,
• an extract of *Acacia macrostachya* seeds, an extract of maca leaves, an extract of *Schisandra sphenanthera* and an extract of *Vigna unguiculata* seeds,
• oxazolines, preferably 2-undecyl-4-hydroxymethyl-4-methyl-1,3-oxazoline, 2-undecyl-4,4-dimethyl-1,3-oxazoline, (E)-4,4-dimethyl-2-heptadec-8-enyl-1,3-oxazoline, 4-hydroxymethyl-4-methyl-2-heptadecyl-1,3-oxazoline, (E)-4-hydroxymethyl-4-methyl-2-heptadec-8-enyl-1,3-oxazoline, 2-undecyl-4-ethyl-4-hydroxymethyl-1,3-oxazoline and 2-undecyl-4,4-dimethyl-1,3-oxazoline, and
mixtures thereof.

9. The composition of any one of claims 5 to 8, **characterized in that** it is formulated to be administered via the topical or oral route.

10. The composition of any one of claims 5 to 9 or extract as defined in any one of claims 1 to 4 to be used in the prevention and/or treatment of disorders or pathologies of the skin and/or mucous membranes and/or skin appendages, advantageously allergic, inflammatory or irritative reactions or pathologies or disorders of the barrier or homeostasis of the skin, or vascular disorders or as depigmenting and healing agent.

11. A cosmetic care method for the skin and/or skin appendages and/or mucous membranes, advantageously for sensitive skin, with a view to improving the condition and/or appearance thereof, in particular in order to promote the hydration thereof, or to improve the firmness, elasticity or tonicity of the skin and to prevent the accumulation of adipose tissue and skin with cellulitis, or to reduce pigmentation spots, or to prevent and/or treat aging, comprising the administration of a cosmetic composition according to any one of claims 5 to 9 or an extract as defined in any one of claims 1 to 4.
